# EUROPEAN PATENT SPECIFICATION

(11) **EP 3 440 089 B1**
(45) Date of publication and mention of the grant of the patent: **29.01.2025**
(21) Application number: 17710560.8
(22) Date of filing: 16.03.2017
(51) Int. Cl.: C07H 19/20, C07F 9/6561, A23L 3/40, A23L 5/00, A23L 23/10, A23L 31/15, A23L 29/00, A23L 29/269, A23L 29/30, A23L 27/00, A23L 27/10, A23L 27/40, A23L 33/13, A23L 27/14, A23L 27/23, A23L 27/50, A23P 10/40, A23P 10/43, A23L 5/20, A23L 31/00

(54) **DEHYDRATED NA2-IMP AS ANTI-CAKING AGENT**
DEHYDRATISIERTES NA2-IMP ALS ANTIBACKMITTEL
NA2-IMP DÉSHYDRATÉ COMME AGENT ANTI-AGGLOMÉRANT

(30) Priority: 08.04.2016 EP 16164489
(43) Date of publication of application: 13.02.2019
(73) Proprietor: Société des Produits Nestlé S.A., 1800 Vevey (CH)
(72) Inventor: FORNY, Laurent, 1000 LAUSANNE 26 (CH); NG, Yun Ting Sherrilyn, SINGAPORE 120112 (SG); ULMER, Helge, 78224 SINGEN (DE)
(74) Representative: Dinger, Hansjörg
(86) International application number: PCT/EP2017/056239
(87) International publication number: WO 2017/174322

(56) References cited:
- EP-A2- 1 424 341
- WO-A2-2011/000824
- ANONYMOUS: "Inosine-5'-monophosphate Disodium Salt Hydrate - Safety Data Sheet", 26 September 2017 (2017-09-26), https://www.trc-canada.com, pages 1 - 6, XP055683353, Retrieved from the Internet <URL:https://web.archive.org/web/20200406131325if_/https://www.trc-canada.com/prod-img/MSDS/I665005MSDS.pdf> [retrieved on 20200406]
- ANONYMOUS: "Guanosine 5'-Monophosphate Disodium Salt - Catalogue Number: S634100", 20 December 2019 (2019-12-20), www.trc-canada.com/, XP055654456, Retrieved from the Internet <URL:https://web.archive.org/web/20191220075632/https://www.trc-canada.com/product-detail/?S634100> [retrieved on 20191220]
- ANONYMOUS: "Hygroscopy - Wikipedia", 9 February 2016 (2016-02-09), pages 1 - 4, XP055683335, Retrieved from the Internet <URL:https://en.wikipedia.org/w/index.php?title=Hygroscopy&oldid=704153345> [retrieved on 20200406]
- LOWRY MARTIN T ET AL: "The properties of powders. Part I. - The caking of salts", TRANS. FARADAY SOC., vol. 15, January 1919 (1919-01-01), pages 1 - 28, XP093062742, Retrieved from the Internet <URL:https://pubs.rsc.org/en/content/articlelanding/1919/TF/TF91915BB001> DOI: 10.1039/TF91915BB001
- YACOUBOU JEANNE: "Disodium Inosinate and Disodium Guanylate Are All-Vegetable Flavor Enhancers", THE VEGETARIAN RESOURCE GROUP BLOG, 21 March 2011 (2011-03-21), www.vrg.org, pages 1 - 4, XP093063355, Retrieved from the Internet <URL:https://www.vrg.org/blog/2011/03/21/disodium-inosinate-and-disodium-guanylate-are-all-vegetable-flavor-enhancers/> [retrieved on 20230712]
- S. T. RAO ET AL: "Crystal and molecular structure of sodium inosine 5'-monophosphate, and anticodon nucleotide of transfer ribonucleic acid", CHEMICAL COMMUNICATIONS., vol. 0, no. 16, 1 January 1968 (1968-01-01), GB, pages 995 - 996, XP055284136, ISSN: 0009-241X, DOI: 10.1039/C19680000995
- ALI NOKHODCHI: "An Overview of the Effect of Moisture on Compaction and Compression States of water in a powder", PHARMACEUTICAL TECHNOLOGY JANUARY, 1 January 2005 (2005-01-01), XP055151101, Retrieved from the Internet <URL:http://www.pharmtech.com/pharmtech/data/articlestandard/pharmtech/022005/141826/article.pdf> [retrieved on 20141105]

## Description

The present invention relates to the field of powders, particularly food powders, and to the avoidance of caking in such powders by using compositions comprising a dehydrated or partially dehydrated disodium inosine monophosphate salt.

Caking of powders, particularly of food powders that occurs during mixing, storage, handling and packaging of such powders, is a common problem to the industry. Caking or lumping is understood, when a food powder forms lumps or agglomerated masses during storage and processing, rather than to flow or continue to flow smoothly as a free flowing powder. Most of the time caking of dry or dehydrated powder is undesired and contributes to reduced product quality, product homogeneity, poor rehydration and dispensability, deterioration of organoleptic quality and shortened shelf life.

Many food powders, whether ingredients, intermediate or final products, are sensitive to caking leading to obvious operational and quality issues. For instance, caked powders cannot be properly discharged in automated industrial installations e.g. for packaging purposes or from final packaging materials to be dosed by consumers. This risk of caking is especially true for sensitive amorphous culinary powders such as tomato powder, bio-hydrolysates, or onion powder, as well as some crystalline powders

Dry mixing of food-grade anti-caking agents is a common option to avoid caking of food powders. Examples of anti-caking agents known in the prior art are wheat flour, native starch, carbonates of calcium, phosphates (tri-calcium phosphate), silicon dioxide, calcium silicate, and others. However, not all of these agents are very efficient and/or positively perceived by consumers. Furthermore, not all of those known anti-caking agents can be applied to every food powder as they may have some other further functionalities potentially affecting the organoleptic and/or textural properties of a final food product, as e.g. with the use of wheat flour or starches.

For example, JP2000233922 describes a method to prevent caking of hygroscopic inorganic powders by mixing burnt alum by 0.1-5 wt. % into one or more kinds of low water content hygroscopic inorganic powders selected from salts consisting essentially of sodium chloride such as salt for food, ammonium sulphate, ammonium chloride and potassium chloride, which contains about 0.1 wt. % moisture. The burnt alum absorbs the moisture in the atmosphere such as air to constantly keep the hygroscopic inorganic powder to a dried state.

US2238149 describes a process for reducing the caking tendency of hygroscopic, pulverulent compounds by thoroughly mixing with substantially anhydrous calcium citrate in quantities sufficient to substantially reduce the caking tendency. The calcium citrate is produced through crystalline citric acid and addition of small amounts of calcium oxide.

EP1424341A2 relates to a method for producing crystals of purine nucleotide disodium derivatives, including 5'-inosinic acid disodium crystals, which are important as flavourings and pharmaceutical products. The method removes the remaining alcohol such as methanol or ethanol from crystals in a short time, thereby preventing the agglomeration of crystals. The method comprises overdrying purine nucleotide derivative disodium crystals containing an alcohol and bringing the overdried crystals into contact with an aqueous solution containing a hydrophilic organic solvent, to control the humidity of the crystals. The overdrying step may dry the purine nucleotide derivative disodium crystals with the controlled humidity to a hydrate equivalent water content of at most 5 hydrates.

It would hence be desirable in the art and the food industry in general to find alternatives and/or even better solutions for compounds which can be used in food powders to reduce or even prevent caking during storage, mixing, handling and/or processing.

### Summary of the invention

The object of the present invention is to improve the state of the art and to provide an improved or at least an alternative solution to overcome at least some of the inconveniences described above.

Particularly, the object of the present invention is to provide a new use of a compound which has an excellent anti-caking property in powders, and particularly in food powders comprising a hygroscopic material, and which is safe to be consumed by humans.

A further object of the present invention is to provide the art with a new use of an anti-caking compound which is safe to be consumed and which as a compound in solubilized form is well recognized and accepted by most consumers.

The object of the present invention is achieved by the subject matter of the independent claims. The dependent claims further develop the idea of the present invention.

Accordingly, the present disclosure provides in a first aspect a disodium inosine-5'-monophosphate (Na₂-5'-IMP) × (n·H₂O) salt, wherein n is selected from 0.0 to 7.0, preferably from 0.5 to 6.5.

In a second aspect, the invention relates to a composition comprising the said disodium inosine-5'-monophosphate salt, as defined in independent claim 1.

A third aspect of the invention relates to the use of the said disodium inosine-5'-monophosphate salt for reducing caking of a composition, the composition comprising at least one hygroscopic material, as defined in independent claim 9. Further aspects of the present invention pertain to methods for reducing caking of a composition which comprises at least one hygroscopic material, as defined in independent claims 10 and 11. One method comprises the step of adding dehydrated or partially dehydrated disodium inosine-5'-monophosphate salt to said composition. Another method comprises first adding a disodium inosine-5'-monophosphate salt to said composition and then dehydrating the composition at a temperature of at least 70°C for at least 3 minutes.

A still further method for reducing caking of a composition comprising disodium inosine-5'-monophosphate salt and at least one hygroscopic material, pertains to the method comprising the step of dehydrating or partially dehydrating the disodium inosine-5'-monophosphate salt in an atmosphere of relative humidity of 2% or below.

The inventors have found that by dehydrating compositions comprising disodium inosine monophosphate (Na₂-5'-IMP), an ingredient commonly added to food powders in non-dehydrated form as a flavour enhancer for umami-ness, the need for additional anti-caking agents can be avoided. Therefore, surprisingly, dehydrated or partially dehydrated disodium inosine monophosphate can be used to replace conventionally used other anti-caking agents to effectively avoid caking of food powders.

Disodium inosine-5' monophosphate, also known as disodium inosinate (E361), is usually commercially available in the hydrated crystalline state with 7.5 water molecules per inosine monophosphate molecule, i.e. as C₁₀H₁₁O₈N₄PNa₂·7.5H₂O (molecular weight of 527.25). It is possible to partially or completely dehydrate the disodium inosine-5' monophosphate by heating it to a temperature of above the critical temperature of water release for few minutes up to several hours depending on the amount and the technology used. Several technologies can be used for such dehydration, for example drying in an oven, a tumbler, or using a double jacketed mixer, vacuum drier or fluid bed drying system. Alternatively, it is also possible to partially or completely dehydrate the disodium inosine-5' monophosphate by flushing it with a low or no-moisture containing gas, such as e.g. nitrogen, for a certain period at room temperature or above.

If compositions comprising hydrated disodium inosine-5' monophosphate (Na₂-5'-IMP) are partially dehydrated by simple drying or if the disodium inosine monophosphate is provided already in dehydrated or partially dehydrated form, they will have the tendency to very quickly re-absorb moisture from the environment, even at low relative humidity. Therefore, the inventors have found that if compositions comprising disodium inosine monophosphate are combined in the dehydrated form with other food powders, they will tend to absorb the moisture from these ingredients very rapidly and therefore protect them from caking. The process described in the present invention was particularly effective if the food powder to be combined with the compositions comprising dehydrated or partially dehydrated Na₂-5'-IMP powder has a glass transition temperature below the storage temperature.

Remarkably, this effect was not observed for other hydrated compounds. For example, monosodium glutamate (MSG) monohydrate (another food flavour enhancer), is itself caking upon dehydration and is not easily reabsorbing moisture. This makes it inefficient for use as an anti-caking agent. Conversely, anhydrous dextrose will only re-absorb moisture above 85% relative humidity.

### Brief Description of the Drawings

Figure 1: Determination of free-flowing capability of tomato powders alone or in combination with fully hydrated and partially dehydrated IMP, after storage at 25°C (A), and 40°C (B) for 2h. 1) is tomato powder alone; 2) is tomato powder with hydrated Na₂-IMP; and 3) is tomato powder with dehydrated Na₂-IMP.
Figure 2: Determination of free-flowing capability of soy sauce powder (SSP) alone or in combination with fully hydrated and partially dehydrated Na₂-IMP at 30sec and 1 to 15 min drying at 90°C, after storage at 25°C (A) and 40°C (B) for 2 hours.
Figure 3: Mixtures of a flavour mix comprising Na₂-IMP with commercial dry tomato powder in the ratios as indicated after storing the mixtures at 60°C for 180 min. The tubes were inverted to show caking or not.

### Detailed Description of the invention

In a fist aspect the present disclosure pertains to a disodium inosine-5'-monophosphate (Na₂-5'-IMP) × (n·H₂O) salt, wherein n is selected from 0.0 to 7.0. Preferably n is selected from 0.5 to 6.5. N can also be selected for being smaller than 6.0, or even smaller than 5.0. Advantageously, n is selected to be above 0.7. As evidence is provided here below in the Example section, anti-caking properties of partially dehydrated Na₂-5'-IMP can already be determined when the corresponding salts have a water content of n equal 7.0 or just below. Significant anti-caking results are then detected with Na₂-5'-IMP salts having a water content of n equal 6.5 or below. Preferably, the water content of the salt is n equal 6.0 or below, more preferably 5.5 or below, even more preferably 5.0 or even 4.5 or below. Excellent results can be achieved when n is equal 4.0, 3.5 or 3.0 or below. Drying the Na₂-5'-IMP salt can get very long and expensive when trying to get rid of all captured water molecules in the salt. Therefore, advantageously, the Na₂-5'-IMP salt still contains water in an amount of n equal 0.5, 0.7, 0.8, 1.2 or above said amounts.

In a second aspect, the invention relates to a composition comprising a disodium inosine-5'-monophosphate (Na₂-5'-IMP) × (n·H₂O) salt, wherein n is selected from 0.0 to 6.5, and wherein the composition further comprises a hygroscopic material, wherein the hygroscopic material is selected from the group consisting of crystalline fructose, crystalline table salt, crystalline malic acid, yeast extract powder, soy sauce powder, a dehydrated vegetable powder, a dehydrated meat powder, a dehydrated plant hydrolysate, a dehydrated meat hydrolysate, or a combination therefrom, and wherein the composition comprises at least 2 wt% of the disodium inosine-5'-monophosphate salt.

In a preferred embodiment of the composition according to the invention, the disodium inosine-5'-monophosphate salt is in crystalline form.

In a preferred embodiment, said composition of the present invention comprises preferably at least 2.5 wt%, more preferably at least 5 wt% of the dehydrated or partially dehydrated disodium inosine-5'-monophosphate salt. In one embodiment of the present invention, the composition comprises at least 7.5 wt%, at least 10 wt%, or at least 20 wt% of the dehydrated or partially dehydrated disodium inosine-5'-monophosphate salt. Wt% pertains to the weight per cent of dry or dried ingredients of the compositions.

The composition according to the present invention further comprises a hygroscopic material. In one embodiment, the composition of the present invention comprises at least 10 wt%, preferably at least 15 wt%, more preferably at least 20 wt% of the hygroscopic material. The composition may comprise at least 30 wt% or at least 40 wt% of the hygroscopic material. The composition may also comprise at least 50 wt%, at least 60 wt%, or at least 70 wt% of the hygroscopic material. The hygroscopic material may be composed of crystalline and/or amorphous material.

The hygroscopic material is selected from the group consisting of crystalline fructose, crystalline table salt, crystalline malic acid, yeast extract powder, soy sauce powder, a dehydrated vegetable powder, a dehydrated meat powder, a dehydrated plant hydrolysate, a dehydrated meat hydrolysate, or a combination therefrom.

In one embodiment, the composition of the present invention is dehydrated. Dehydrated composition means that the composition is substantially water free and dry. The composition has been assembled from either dry materials and/or wet materials which have been dried before or during assembly.

In a preferred embodiment, the composition of the present invention is food-grade. Food-grade means that the composition is safe for being consumed by a human and/or animal.

Preferably, the composition of the present invention is a food seasoning, a food condiment, a soup concentrate or a sauce concentrate. Preferably, these food products are in a dehydrated form.

In an embodiment, the composition of the present invention is in the form of a powder, a granulated product or a tablet. Particularly and preferably, the composition of the present invention is a food condiment, a soup concentrate or a sauce concentrate in the form of a powder, a granulated product or a tablet.

A further aspect of the present invention pertains to the use of a disodium inosine-5'-monophosphate (Na₂-5'-IMP) x (n-H₂O) salt, wherein n is selected from 0.0 to 6.5, for reducing caking of a composition, the composition comprising at least one hygroscopic material, wherein the hygroscopic material is selected from the group consisting of crystalline fructose, crystalline table salt, crystalline malic acid, yeast extract powder, soy sauce powder, a dehydrated vegetable powder, a dehydrated meat powder, a dehydrated plant hydrolysate, a dehydrated meat hydrolysate, or a combination therefrom, and wherein the composition comprises at least 2 wt% of the disodium inosine-5'-monophosphate salt.

Furthermore, the present invention also pertains to a method for reducing caking of a composition which comprises at least one hygroscopic material, the method comprising the step of adding disodium inosine-5'-monophosphate (Na₂-5'-IMP) x (n H₂O) salt, wherein n is selected from 0.0 to 6.5, to said composition, wherein the hygroscopic material is selected from the group consisting of crystalline fructose, crystalline table salt, crystalline malic acid, yeast extract powder, soy sauce powder, a dehydrated vegetable powder, a dehydrated meat powder, a dehydrated plant hydrolysate, a dehydrated meat hydrolysate, or a combination therefrom, and wherein the composition comprises at least 2 wt% of the disodium inosine-5'-monophosphate salt.

The present invention also pertains to a method for reducing caking of a composition which comprises at least one hygroscopic material, the method comprising the step of first adding a disodium inosine-5'-monophosphate salt to said composition and then dehydrating the composition at a temperature of at least 70°C for at least 3 minutes, wherein the hygroscopic material is selected from the group consisting of crystalline fructose, crystalline table salt, crystalline malic acid, yeast extract powder, soy sauce powder, a dehydrated vegetable powder, a dehydrated meat powder, a dehydrated plant hydrolysate, a dehydrated meat hydrolysate, or a combination therefrom, and wherein the composition comprises at least 2 wt% of the disodium inosine-5'-monophosphate salt.

The disodium inosine-5'-monophosphate salt added first to the composition may be of dehydrated, partially dehydrated or regular, non-dehydrated Na₂-5'-IMP salt. Preferably, the composition is dehydrated at a temperature of at least 70°C, at least 80°C or at least 85°C for 5 min, 10 min, 15 min, 30 min or above. This allows good dehydration results of the Na₂-5'-IMP salt, which then results in excellent anti-caking properties of said salt.

A still further method for reducing caking of a composition comprising disodium inosine-5'-monophosphate salt and at least one hygroscopic material, comprises the step of dehydrating or partially dehydrating the disodium inosine-5'-monophosphate salt in an atmosphere of relative humidity of 2% or below. Preferably, the atmosphere has a relative humidity of below 1.5%, and even more preferably the relative humidity of the atmosphere is close to 0%. Most preferably, the atmosphere is a dry nitrogen gas. The dehydration step of this method can be conducted at an ambient room temperature or above. Preferably, the dehydration step is for at least 1 hour, preferably for at least 2 hours, more preferably for at least 4 hours. The disodium inosine-5'-monophosphate salt can be dehydrated or partially dehydrated either before being added to the composition or after being added to the composition.

Those skilled in the art will understand that described for the products of the present invention may be combined with the use and methods of the present invention and vice versa. Further, features described for different embodiments of the present invention may be combined. Further advantages and features of the present invention are apparent from the figures and examples.

### Example 1: Screening of dehydrated salts for anti-caking properties (only the mixture of partially or completely dehydrated Na₂-5'-IMP with commercial dry tomato powder in a ratio of 1:2 is according to the present invention)

Different food-grade salts as typically used in the culinary food products were tested for anti-caking activity when dehydrated or partially dehydrated before mixing them together with standard commercial dry tomato powder. The experimental set-up was as follows:
i) the salts were partially or completely dehydrated by placing them in an oven at 90°C for 2 hours;
ii) the salts were then mixed with commercial dry tomato powder in a ratio of 1:2. The dehydrated salts as mentioned above were tested in parallel versus non-dehydrated salts in the same experimental set-up;
iii) the mixtures were then immediately put into tubes (about 4 grams per sample tube), sealed and stored for 2 hours at 25°C and 40°C, respectively;
iv) occurrence of caking was then visually assessed by turning the tubes upside down.

No caking occurs where the powder mix remains free-flowing and readily moves down the tube upon turning the tubes upside down. Caking was observed where the powder mix was not free-flowing anymore and remained agglomerated (i.e. caked) on the bottom of the tube upon turning the tube upside down.

The results can be summarized as follows:
CaCl₂·2H₂O and Na₂-IMP·7.5H₂O showed the best results of anti-caking after partial dehydration in comparison to all other salts tested. Other salts tested such as calcium carbonate, monosodium glutamate (MSG), MgCl₂·6H₂O, citric acid, and D-maltose only showed very weak or no anti-caking activity after the dehydration step.

Further analysis of the anhydrous CaCl₂ and Na₂-IMP salts revealed that anhydrous CaCl₂ is only efficient from 0 up to about 22% relative humidity of reabsorption, where the salt then starts to become liquid. Anhydrous Na₂-IMP salt, however, is efficient from 0 to above 95% relative humidity. Consequently, anhydrous Na₂-IMP salt has a very broad range of moisture absorbance, and is therefore much more efficient and versatile when it comes to usability of the salt as a natural food-grade anti-caking agent when for example compared to CaCl₂ or other known absorbent salts. For example anhydrous dextrose is known to re-absorb moisture only above ca. 85% relative humidity.

### Example 2: Na₂-IMP as anti-caking agent

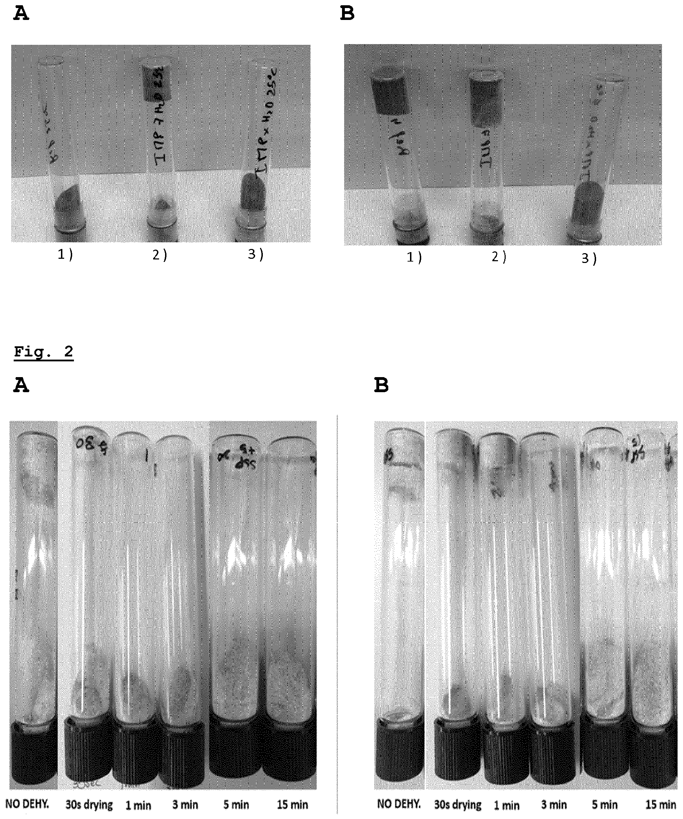

Na₂-IMP·7.5H₂O was partially dehydrated by placing it in an oven at 90°C for 2 hours (see Table 1). Tomato powder was then dry mixed with the different forms of IMP - fully hydrated (7.5H₂O) as negative control and partially dehydrated (0.78H₂O) at a ratio of 66:33. The dry mix (about 4 grams per tube) was stored in tubes at 25°C or 40°C for 2h. The caking occurrence was visually assessed by turning the tubes upside down (see Figure 1). Water activity of the mixtures before and after the storage tests was then also determined. It was observed that when the IMP is partially dehydrated, it takes moisture from the tomato powder as evidenced by a lowering of the water activity from Aw 0.21 (mixed with fully hydrated IMP) to Aw 0.08 (partially dehydrated IMP) at 25°C, and from Aw 0.21 (mixed with fully hydrated IMP) to Aw 0.09 (partially dehydrated IMP) when stored at 40°C. As a consequence, the powder remains free-flowing and able to move down the tube.

**Table 1: Determining level of dehydration for IMP samples:**

| | |
|---|---|
| Empty dish (g) | 110.25 |
| Dish with sample (g) | 123.76 |
| Moisture loss (%) | 21.61 |
| Assumed initial hydration | 7.00 |
| MW (g/mol) | 392.17 |
| Estimated final hydration | 0.78 |

### Example 3: Test of NaCl for anti-caking properties (not according to the present invention)

Anhydrous NaCl was dry mixed with tomato powder at a ratio of 50:50. The dry mix was then stored in tubes at 50⁰C. The caking occurrence was visually assessed by turning the tubes upside down. It was observed that anhydrous crystals such as NaCl does not prevent caking as the tomato powder/NaCl mixture was not able to move freely down the tube (Results not shown).

### Example 4: Test of mix of ribonucleotides for anti-caking properties (not according to the present invention)

Ribonucleotides (50% IMP and 50% GMP) were partially dehydrated by placing them in an oven at 90°C for 3 hours. Crystalline malic acid was then dry mixed with the different forms of ribonucleotides - fully hydrated and partially dehydrated at a ratio of 50:50. The dry mix was then stored in tubes at 25°C or 40°C for 2h as in the previous examples. The caking occurrence was visually assessed by turning the tubes upside down. The result indicates that when the ribonucleotides are partially dehydrated, they take up moisture from the environment in the tube as evidenced by a lowering of the water activity from Aw 0.665 (mixed with fully hydrated ribonucloetides) to Aw 0.059 (partially dehydrated ribonucleotides) at 25°C, and from Aw 0.453 (mixed with fully hydrated ribonucleotides) to Aw 0.01 (partially dehydrated ribonucleotides) when stored at 40°C. As a consequence, the powder with the dehydrated ribonucleotides and the malic acid remains free-flowing and able to move down the tube, while the powder with the hydrated normal ribonucleotides and the malic acid caked on the bottom of the tube and was not free flowing anymore.

### Example 5: Test of mix of ribonucleotides for anti-caking properties (not according to the present invention)

Ribonucleotides (50% IMP & 50% GMP) were partially dehydrated by placing them in an oven at 90°C for 3 hours. Amorphous soya sauce powder (SSP) was then dry mixed with the different forms of the ribonucleotides - fully hydrated and partially dehydrated at a ratio of 50:50. The dry mix was then stored in tubes at 25°C or 40°C as indicated in the previous Examples. The caking occurrence was visually assessed by turning the tubes upside down. The results indicate that when the ribonucleotides are partially dehydrated, they take up moisture from the SSP as evidenced by a lowering of the water activity from Aw 0.265 (mixed with fully hydrated ribonucleotides) to Aw 0.03 (partially dehydrated ribonucleotides) at 25°C, and from Aw 0.235 (mixed with fully hydrated ribonucleotides) to Aw 0.03 (partially dehydrated ribonucleotides) when stored at 40°C. As a consequence, the powder with the dehydrated ribonucleotides remains free-flowing and able to move down the tube, while the powder with the hydrated normal ribonucleotides caked on the bottom of the tube and was not free flowing anymore.

### Example 6: Na₂-IMP as anti-caking agent (only the dry mixes at a 50:50 ratio between soy sauce powder and Na₂-IMP, wherein the Na₂-IMP has been dehydrated at 90°C for 3 min or longer, are according to the present invention)

Na₂-IMP·7.5H₂O was partially dehydrated by placing it in an oven at 90°C for different time periods as specified in Table 2. Soy Sauce Powder (SSP) was then dry mixed with the different forms of Na₂-IMP, fully hydrated (7.5H₂O) as negative control and partially dehydrated forms as specified in the Table below, at a ratio of 50:50. The dry mixes were then stored in tubes at 25°C or 40°C for 2h as described in the previous Examples. The caking occurrence was visually assessed by turning the tubes upside down. It can be seen that when the Na₂-IMP is partially dehydrated, it takes moisture from the SSP as evidenced by a lowering of the water activity. The results of the experiment are summarized in Table 3 and shown in Figure 2.

**Table 2: Determining level of dehydration for Na₂-IMP sample**

| Sample | Empty Dish (g) | Dish with sample (g) | After drying (g) | Moist ure loss (%) | MW (g/mol) | Estimated final hydration | Aw |
|---|---|---|---|---|---|---|---|
| 30 sec | 1.1845 | 4.8168 | 4.8049 | 0.33 | 392.17 | 6.91 | 0.4311 |
| 1 min | 45.7163 | 53.2087 | 53.1656 | 0.58 | 392.17 | 6.83 | |
| 3 min | 1.1889 | 4.893 | 4.8228 | 1.90 | 392.17 | 6.45 | 0.2730 |
| 5 min | 45.7121 | 54.9576 | 54.7926 | 1.78 | 392.17 | 6.49 | |
| 15 min | 46.8126 | 53.9445 | 53.5755 | 5.17 | 392.17 | 5.51 | 0.2536 |
| 30 min | 47.0985 | 53.1024 | 52.5001 | 10.03 | 392.17 | 4.11 | |
| 1 h | 52.1889 | 57.0712 | 56.1281 | 19.32 | 392.17 | 1.44 | 0.0691 |
| 2 h | 47.5963 | 51.7259 | 50.8387 | 21.48 | 392.17 | 0.82 | 0.0611 |
| 3 h | 110.25 | 123.76 | 120.84 | 21.61 | 392.17 | 0.78 | 0.056 |

**Table 3: Results of the caking test in the test tubes:**

| Samples | After storage at 25°C for 2h | After storage at 40°C for 2h |
|---|---|---|
| control | caking | caking |
| 30 sec | Slight caking | caking |
| 1 min | Slight caking | caking |
| 3 min | No caking | No caking |
| 5 min | No caking | No caking |
| 15 min | No caking | No caking |

Control: sample with non-dehydrated Na₂-IMP·7.5H₂O

### Example 7: Flavor mix comprising Na₂-IMP (only the mixtures of commercially available dry tomato powder prepared with 10 wt% or more of the dehydrated flavour mix, are according to the present invention)

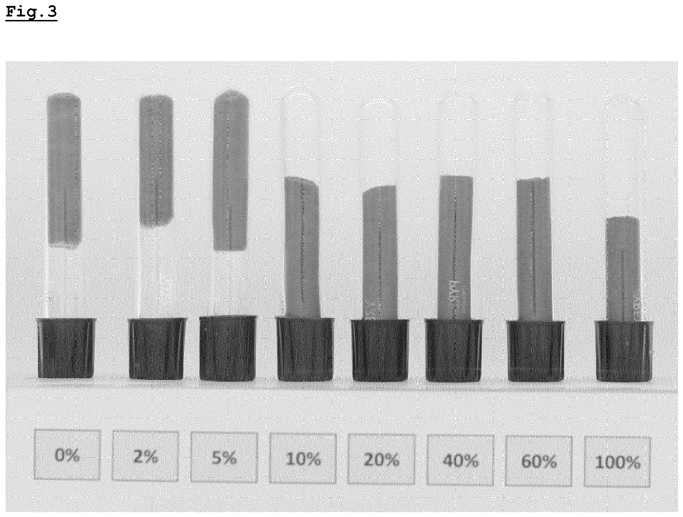

A dry flavour mix obtained via natural fermentation, reconstitution and concentration, comprising natural Na₂-IMP was tested for anti-caking properties. The flavour mix comprised about 22.6 wt% Na₂-IMP·7.5H₂O, 41 wt% NaCl, and the rest being amorphous flavor substances such as mainly simple sugars, peptides and amino acids. The flavour mix was dehydrated in a vacuum dryer at 85°C for 6 hours. Immediately after drying, mixtures of the dehydrated flavour mix were prepared with commercially available dry tomato powder comprising 0, 2, 5, 10, 20, 40, 60 and 100 wt% of the flavour mix, respectively, the rest being tomato powder. About 4 grams of each mixture was then filled into glass tubes and closed air-tight. The tubes were then stored in an oven at 60°C for 180 min and assessed thereafter manually for caking of the mixture by gentle movement and inverting the tubes upside down. The results are shown in Figure 3.

From those results it can be concluded that the presence of the dehydrated flavor mix was sufficient to already significantly prevent caking of a tomato powder at an amount of 10 wt% flavor mix or more. 10 wt% flavor mix comprises ca. 2.26 wt% of dehydrated Na₂-IMP. Consequently, 2.26 wt% or more of dehydrated Na₂-IMP were sufficient to prevent caking of a total composition comprising at least 90 wt% of a hygroscopic material, i.e. of dry tomato powder.

## Claims

1. A composition comprising a disodium inosine-5'-monophosphate (Na₂-5'-IMP) x (n·H₂O) salt, wherein n is selected from 0.0 to 6.5, and wherein the composition further comprises a hygroscopic material,
wherein the hygroscopic material is selected from the group consisting of crystalline fructose, crystalline table salt, crystalline malic acid, yeast extract powder, soy sauce powder, a dehydrated vegetable powder, a dehydrated meat powder, a dehydrated plant hydrolysate, a dehydrated meat hydrolysate, or a combination therefrom, and wherein the composition comprises at least 2 wt% of the disodium inosine-5'-monophosphate salt.

2. The composition according to claim 1, wherein the disodium inosine-5'-monophosphate salt is in crystalline form.

3. The composition according to claim 1 or 2, wherein the composition comprises at least 2.5 wt%, more preferably at least 5 wt% of the disodium inosine-5'-monophosphate salt.

4. The composition according to one of the claims 1-3, wherein the composition comprises at least 10 wt%, preferably at least 15 wt%, more preferably at least 20 wt% of the hygroscopic material.

5. The composition according to one of the claims 1-4, wherein the composition is dehydrated.

6. The composition according to one of the claims 1-5, wherein the composition is food-grade.

7. The composition according to claim 6, wherein the composition is a food seasoning, a food condiment, a soup concentrate or a sauce concentrate.

8. The composition according to one of the claims 1-7, which is in the form of a powder, a granulated product or a tablet.

9. Use of a disodium inosine-5'-monophosphate (Na₂-5'-IMP) x (n·H₂O) salt, wherein n is selected from 0.0 to 6.5, for reducing caking of a composition, the composition comprising at least one hygroscopic material,
wherein the hygroscopic material is selected from the group consisting of crystalline fructose, crystalline table salt, crystalline malic acid, yeast extract powder, soy sauce powder, a dehydrated vegetable powder, a dehydrated meat powder, a dehydrated plant hydrolysate, a dehydrated meat hydrolysate, or a combination therefrom, and wherein the composition comprises at least 2 wt% of the disodium inosine-5'-monophosphate salt.

10. Method for reducing caking of a composition which comprises at least one hygroscopic material, the method comprising the step of adding disodium inosine-5'-monophosphate (Na₂-5'-IMP) x (n·H₂O) salt, wherein n is selected from 0.0 to 6.5, to said composition,
wherein the hygroscopic material is selected from the group consisting of crystalline fructose, crystalline table salt, crystalline malic acid, yeast extract powder, soy sauce powder, a dehydrated vegetable powder, a dehydrated meat powder, a dehydrated plant hydrolysate, a dehydrated meat hydrolysate, or a combination therefrom, and wherein the composition comprises at least 2 wt% of the disodium inosine-5'-monophosphate salt.

11. Method for reducing caking of a composition which comprises at least one hygroscopic material, the method comprising the step of first adding a disodium inosine-5'-monophosphate salt to said composition and then dehydrating the composition at a temperature of at least 70°C for at least 3 minutes,
wherein the hygroscopic material is selected from the group consisting of crystalline fructose, crystalline table salt, crystalline malic acid, yeast extract powder, soy sauce powder, a dehydrated vegetable powder, a dehydrated meat powder, a dehydrated plant hydrolysate, a dehydrated meat hydrolysate, or a combination therefrom, and wherein the composition comprises at least 2 wt% of the disodium inosine-5'-monophosphate salt.

## Patentansprüche

1. Zusammensetzung, umfassend ein Salz von Dinatriuminosin-5'-monophosphat (Na₂-5'-IMP) x (n·H₂O), wobei n aus 0,0 bis 6,5 ausgewählt ist, und wobei die Zusammensetzung ferner ein hygroskopisches Material umfasst, wobei das hygroskopische Material aus der Gruppe ausgewählt ist, die aus kristalliner Fructose, kristallinem Speisesalz, kristalliner Apfelsäure, Hefeextraktpulver, Sojasoßenpulver, einem dehydratisierten Gemüsepulver, einem dehydratisierten Fleischpulver, einem dehydratisierten Pflanzenhydrolysat, einem dehydratisierten Fleischhydrolysat oder einer Kombination davon besteht, und wobei die Zusammensetzung mindestens zu 2 Gew.-% Dinatriuminosin-5'-monophosphatsalz umfasst.

2. Zusammensetzung nach Anspruch 1, wobei das Dinatriuminosin-5'-monophosphatsalz in kristalliner Form vorliegt.

3. Zusammensetzung nach Anspruch 1 oder 2, wobei die Zusammensetzung zu mindestens 2,5 Gew.-%, noch bevorzugter zu mindestens 5 Gew.-%, Dinatriuminosin-5'-monophosphatsalz umfasst.

4. Zusammensetzung nach einem der Ansprüche 1 bis 3, wobei die Zusammensetzung zu mindestens 10 Gew.-%, vorzugsweise zu mindestens 15 Gew.-%, noch bevorzugter zu mindestens 20 Gew.-%, hygroskopisches Material umfasst.

5. Zusammensetzung nach einem der Ansprüche 1 bis 4, wobei die Zusammensetzung dehydratisiert ist.

6. Zusammensetzung nach einem der Ansprüche 1 bis 5, wobei die Zusammensetzung Lebensmittelqualität aufweist.

7. Zusammensetzung nach Anspruch 6, wobei die Zusammensetzung ein Lebensmittelgewürz, ein Lebensmittelwürzmittel, ein Suppenkonzentrat oder ein Soßenkonzentrat ist.

8. Zusammensetzung nach einem der Ansprüche 1 bis 7, die in Form eines Pulvers, eines granulierten Produkts oder einer Tablette vorliegt.

9. Verwendung eines Salzes von Dinatriuminosin-5'-monophosphat (Na₂-5'-IMP) x (n·H₂O), wobei n aus 0,0 bis 6,5 ausgewählt ist, zum Reduzieren des Verklumpens einer Zusammensetzung, wobei die Zusammensetzung mindestens ein hygroskopisches Material umfasst, wobei das hygroskopische Material aus der Gruppe ausgewählt ist, die aus kristalliner Fructose, kristallinem Speisesalz, kristalliner Apfelsäure, Hefeextraktpulver, Sojasoßenpulver, einem dehydratisierten Gemüsepulver, einem dehydratisierten Fleischpulver, einem dehydratisierten Pflanzenhydrolysat, einem dehydratisierten Fleischhydrolysat oder einer Kombination davon besteht, und wobei die Zusammensetzung zu mindestens 2 Gew.-% Dinatriuminosin-5'-monophosphatsalz umfasst.

10. Verfahren zum Reduzieren des Verklumpens einer Zusammensetzung, die mindestens ein hygroskopisches Material umfasst, wobei das Verfahren den Schritt der Zugabe von Dinatriuminosin-5'-monophosphatsalz (Na₂-5'-IMP) x (n·H₂O), wobei n aus 0,0 bis 6,5 ausgewählt ist, zu der Zusammensetzung, wobei das hygroskopische Material aus der Gruppe ausgewählt ist, die aus kristalliner Fructose, kristallinem Speisesalz, kristalliner Apfelsäure, Hefeextraktpulver, Sojasoßenpulver, einem dehydratisierten Gemüsepulver, einem dehydratisierten Fleischpulver, einem dehydratisierten Pflanzenhydrolysat, dehydratisierten Fleischhydrolysat oder einer Kombination davon besteht, und wobei die Zusammensetzung zu mindestens 2 Gew.-% Dinatriuminosin-5'-monophosphatsalz umfasst.

11. Verfahren zum Reduzieren des Verklumpens einer Zusammensetzung, die mindestens ein hygroskopisches Material umfasst, wobei das Verfahren den Schritt umfasst, zunächst ein Dinatriuminosin-5'-monophosphatsalz zu der Zusammensetzung zuzugeben und die Zusammensetzung dann bei einer Temperatur von mindestens 70 °C für mindestens 3 Minuten zu dehydratisieren, wobei das hygroskopische Material ausgewählt ist aus der Gruppe bestehend aus kristalliner Fructose, kristallinem Speisesalz, kristalliner Apfelsäure, Hefeextraktpulver, Sojasoßenpulver, einem dehydratisierten Gemüsepulver, einem dehydratisierten Fleischpulver, einem dehydratisierten Pflanzenhydrolysat, dehydratisierten Fleischhydrolysat oder einer Kombination davon, und wobei die Zusammensetzung zu mindestens 2 Gew.-% das Dinatriuminosin-5'-monophosphatsalz umfasst.

## Revendications

1. Composition comprenant un sel d'inosine-5'-monophosphate disodique (Na₂-5'-IMP) x (n·H₂O), dans laquelle n est choisi entre 0,0 et 6,5, et dans laquelle la composition comprend en outre un matériau hygroscopique, dans laquelle le matériau hygroscopique est choisi dans le groupe constitué de fructose cristallin, sel de table cristallin, acide malique cristallin, poudre d'extrait de levure, poudre de sauce soja, une poudre de légumes déshydratés, une poudre de viande déshydratée, un hydrolysat végétal déshydraté, un hydrolysat de viande déshydratée, ou une combinaison de ceux-ci, et dans laquelle la composition comprend au moins 2 % en poids du sel d'inosine-5'-monophosphate disodique.

2. Composition selon la revendication 1, dans laquelle le sel d'inosine-5'-monophosphate disodique est sous forme cristalline.

3. Composition selon la revendication 1 ou 2, dans laquelle la composition comprend au moins 2,5 % en poids, plus préférablement au moins 5 % en poids du sel d'inosine-5'-monophosphate disodique.

4. Composition selon l'une des revendications 1 à 3, dans laquelle la composition comprend au moins 10 % en poids, de préférence au moins 15 % en poids, plus préférablement au moins 20 % en poids du matériau hygroscopique.

5. Composition selon l'une des revendications 1 à 4, dans laquelle la composition est déshydratée.

6. Composition selon l'une des revendications 1 à 5, dans laquelle la composition est de qualité alimentaire.

7. Composition selon la revendication 6, dans laquelle la composition est un assaisonnement alimentaire, un condiment alimentaire, un concentré de soupe ou un concentré de sauce.

8. Composition selon l'une des revendications 1 à 7, qui se présente sous la forme d'une poudre, d'un produit granulé ou d'un comprimé.

9. Utilisation d'un sel d'inosine-5'-monophosphate disodique (Na₂-5'-IMP) × (n·H₂O), dans laquelle n est choisi entre 0,0 et 6,5, permettant de réduire la prise en masse d'une composition, la composition comprenant au moins un matériau hygroscopique, dans laquelle le matériau hygroscopique est choisi dans le groupe constitué de fructose cristallin, sel de table cristallin, acide malique cristallin, poudre d'extrait de levure, poudre de sauce soja, une poudre de légumes déshydratés, une poudre de viande déshydratée, un hydrolysat végétal déshydraté, un hydrolysat de viande déshydratée, ou une combinaison de ceux-ci, et dans laquelle la composition comprend au moins 2 % en poids du sel d'inosine-5'-monophosphate disodique.

10. Procédé permettant de réduire la prise en masse d'une composition qui comprend au moins un matériau hygroscopique, le procédé comprenant l'étape consistant à ajouter du sel d'inosine-5'-monophosphate disodique (Na₂-5'-IMP) × (n·H₂O), dans lequel n est choisi entre 0,0 et 6,5, à ladite composition, dans lequel le matériau hygroscopique est choisi dans le groupe constitué de fructose cristallin, sel de table cristallin, acide malique cristallin, poudre d'extrait de levure, poudre de sauce soja, une poudre de légumes déshydratés, une poudre de viande déshydratée, un hydrolysat végétal déshydraté, un hydrolysat de viande déshydratée, ou une combinaison de ceux-ci, et dans lequel la composition comprend au moins 2 % en poids du sel d'inosine-5'-monophosphate disodique.

11. Procédé permettant de réduire la prise en masse d'une composition qui comprend au moins un matériau hygroscopique, le procédé comprenant l'étape consistant à ajouter tout d'abord un sel d'inosine-5'-monophosphate disodique à ladite composition, puis à déshydrater la composition à une température d'au moins 70 °C pendant au moins 3 minutes,
dans lequel le matériau hygroscopique est choisi dans le groupe constitué de fructose cristallin, sel de table cristallin, acide malique cristallin, poudre d'extrait de levure, poudre de sauce soja, une poudre de légumes déshydratés, une poudre de viande déshydratée, un hydrolysat végétal déshydraté, un hydrolysat de viande déshydratée, ou une combinaison de ceux-ci, et dans lequel la composition comprend au moins 2 % en poids du sel d'inosine-5'-monophosphate disodique.
